# EUROPÄISCHE PATENTSCHRIFT

(11) **EP 0 277 629 B1**
(45) Veröffentlichungstag und Bekanntmachung des Hinweises auf die Patenterteilung: **05.05.1993**
(21) Anmeldenummer: 88101443.5
(22) Anmeldetag: 02.02.1988
(51) Int. Cl.: G01N 21/90

(54) **Verfahren und Vorrichtung zum Feststellen von Fremdkörpern in Fluiden**
Method and device for detecting impurities in fluids
Procédé et dispositif pour la détection d'impuretés dans des fluides

(30) Priorität: 04.02.1987 DE 3703306
(43) Veröffentlichungstag der Anmeldung: 10.08.1988
(73) Patentinhaber: Harro Höfliger Verpackungsmaschinen GmbH, 71573 Allmersbach im Tal (DE)
(72) Erfinder: Krieg, Gunther, Dipl.-Phys., Prof. Dr., D-7500 Karlsruhe (DE); Barth, Gerhard, Dipl.-Phys., Prof. Dr., D-7500 Karlsruhe 41 (DE); Vaas, Eberhard, Dipl.-Ing., D-7530 Pforzheim (DE); Reiser, Manfred, Dipl.-Ing., D-7057 Winnenden-Hertmannsweiler (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- EP-A- 0 086 143
- DE-A- 3 043 031
- DE-B- 1 573 687
- FR-A- 1 436 128
- US-A- 3 598 907
- US-A- 3 963 348
- US-A- 4 087 184

## Beschreibung

Die Erfindung betrifft ein Verfahren zum Feststellen von Fremdkörpern in Fluiden, insbesondere in in Behältern enthaltenen Flüssigkeiten, wie Infusionslösungen od. dgl., wobei das Fluid flächig mit polarisiertem Licht beleuchtet und das durch das Fluid hindurchtretende Licht im wesentlichen in Richtung der optischen Achse des einfallenden Lichts durch einen zu dessen Polarisationsrichtung gekreuzten Analysator hindurch betrachtet wird, wodurch das durch das Fluid hindurch tretende Licht weitgehend abgedunkelt ist und eine Vorrichtung zum Feststellen von Fremdkörpern in Fluiden, insbesondere von in Behältern enthaltenen Flüssigkeiten, wie Infusionslösungen od. dgl., mit einer Lichtquelle, die das Fluid flächig mit polarisiertem Licht beleuchtet und mit einem im wesentlichen auf der der Lichtquelle abgewandten Seite angeordneten, auf Dunkelfeldbeobachtung eingestellten Analysator.

Bei Infusionsflüssigkeiten, insbesondere in Infusionsflaschen, die eine Menge von mindestens einhundert Millilitern, in der Regel aber mehrere hundert Milliliter und typischerweise einen Liter aufnehmen, stellt sich das Problem, daß in die Infusionsflüssigkeit Fremdteilchen gelangen können, wie beispielsweise Gummi- oder Korkteilchen des Verschlusses, Metallteilchen, Fäder od. dgl.. Derartige Fremdteilchen bergen ab einer gewissen Größe, etwa ab fünfzig Mikrometern die Gefahr, daß sie den Hohlraum einer Infusionsnadel verstopfen. Weiter kann eine Gefährdung des Patienten eintreten, wenn derart relativ große Partikel in die Blutbahn gelangen. Es ist daher notwendig, derartige Infusionsflaschen auf größere Fremdteilchen hin zu untersuchen, so daß Infusionsflaschen mit großen Fremdteilchen aussortiert werden können, während Flaschen mit kleineren Teilchen verbleiben können. Einzelne oder wenige Fremdteilchen über einer bestimmten Größe können auch in anderen Fluiden, worunter hier Flüssigkeiten und Gase verstanden werden, sei es, daß diese in einer Leitung strömen oder aber diskret in Behältnissen, wie Flaschen abgepackt sind, als störend empfunden werden, so daß ein Ausscheidevorgang eingeleitet werden soll.

Es ist bekannt (DE-AS 15 73 687), Ampullen, die aus Glas geschnitten, durch Anschmelzen geformt und verschlossen sind und nur wenig Flüssigkeit, maximal 50 ml aufnehmen, und die in ihnen befindliche Flüssigkeit großflächig über durch Kaltlichtröhren erhellte Leuchtkästen mit Opalglasscheiben über einen vorgeschalteten Polarisationsfilter zu beleuchten und durch einen auf der dem Leuchtkasten abgewandten Seite der Ampullen befindlichen zweiten Polarisationsfilter, der gegenüber dem ersten gekreuzt angeordnet ist, durch eine Person subjektiv beobachten zu lassen, um fehlerhafte Ampullen auszuscheiden. Die subjektive Beobachtung ist für den Beobachter ermüdend und mit erheblichen Unsicherheiten verbunden, und es können nur kleine Gefäße wie eben Ampullen, die darüber hinaus in der beschriebenen Weise aus Glas, guter, optimaler Qualität hergestellt sind, beobachtet werden. Bei Infusionsflaschen, die aus gegossenem Glas bestehen und damit relativ schlechtere optische Qualität aufweisen, führt dieses subjektive Vorgehen zu unvertretbaren Ergebnissen, da einerseits die zu beobachtende Fläche zu groß ist und damit relevante Teilchen im Randbereich übersehen werden, andererseits überraschenderweise Flaschen aufgrund von Glasfehlern unberechtigterweise ausgeschieden werden.

Die DE-OS 31 45 668 schlägt eine Vorrichtung zum optischen Untersuchen von flüssigen Gefäßinhalten auf Fremdkörper mit einem Laser vor, dessen Strahl auf eine zylinderförmige Spiegelfläche gerichtet, von dieser reflektiert und in einer Ebene aufgeweitet wird (Lichtvorhang), der linienförmig die Flüssigkeit beleuchtet, wobei hinter der Flüssigkeit in der durch den Lichtvorhang aufgespannten Ebene eine Reihe von lichtempfindlichen Detektoren angeordnet ist. Ein in den aufgeweiteten Laserstrahl gelangendes Teilchen dunkelt diesen ab, was durch die Fotoelemente als Abweichung von der Normalbeleuchtung festgestellt werden soll. Diese bekannte Vorrichtung lieferte keine zufriedenstellenden Ergebnisse, beispielsweise weil das Meßergebnis nicht teilchengrößenabhängig war und die nach oben und unten entsprechend einer Gaußverteilung abfallende Laserintensität nicht die erforderliche Detektionssicherheit in diesen Bereichen ermöglichte.

In ähnlicher Weise ist bekannt (US-PS 42 74 745) durch einen Spalt ausgeblendetes und durch eine Ampulle hindurchtretendes Licht auf eine Reihe von Detektoren abzubilden und die Intensität zu messen. Hierfür gilt ebenfalls vorstehendes, abgesehen davon, daß nur Gefäße mit kleinem Volumen untersucht werden können. Weiterhin wurde versucht, einen Laserstrahl ebenfalls in einer Ebene als Lichtvorhang aufzuweiten, auf die Infusionsflasche zu richten und Streulicht mittels einer Fernsehkamera aufzunehmen und auszuwerten. Dabei waren Lichtquelle und Kamera nicht fluchtend mit der zu untersuchenden Flüssigkeitsflasche, sondern winklig angeordnet. Dieses Streulichtverfahren ergab keine teilchengrößenabhängigen Meßergebnisse.

Der Erfindung liegt die Aufgabe zugrunde, unter Vermeidung der genannten Nachteile ein Verfahren und eine Vorrichtung zum objektiven Feststellen von Fremdkörpern in größeren Fluidmengen, insbesondere mit größeren Beobachtungsflächen von mehr als 20 cm², vorzugsweise von mehr als 50 cm² zu schaffen, das eine zuverlässige Diskrimination von Fremdteilchen nach ihrer Größe ermöglicht.

Erfindungsgemäß wird die genannte Aufgabe durch ein Verfahren gelöst, welches dadurch gekennzeichnet ist, daß während der Messung der Behälter ruht und die Flüssigkeit in Bewegung ist, daß die Lichtintensität einzelner Bildelemente mittels optoelektrischer Wandlung gemessen wird, daß die Intensitäten mehrerer Bildelemente in einer durch diese gebildeten Meßzone durch Summation oder Mittelung zusammengefaßt werden, daß zeitlich nacheinander mindestens zwei Messungen jeder Meßzone vorgenommen werden und diese für jede Meßzone miteinander verglichen werden und daß bei Abweichung der Intensitätsdifferenz über einen vorgegebenen Schwellwert ein Ausgangssignal erzeugt wird. Eine erfindungsgemäße Vorrichtung sieht zur Lösung der Aufgabe vor, daß während der Messung der Behälter ruht und die Flüssigkeit in Bewegung ist, daß im wesentlichen auf der optischen Achse das auf das Fluid fallende Licht einer Fernsehkamera als Detektionseinrichtung mit in Form einer Matrix angeordneten Bildelementen angeordnet ist, der eine Auswerteeinheit nachgeordnet ist, die eine Einrichtung zum Zusammenfassen der Intensitäten von mehreren einzelnen Bildelementen einer Meßzone zu einem Meßwert durch Summation oder Mittelung aufweist, und daß ein Vergleicher mit einer einstellbaren Schwelle zum Vergleichen der Differenz von zu verschiedenen Zeiten erfaßten, zusammengefaßten Meßwerten einer Meßzone mit der Schwelle vorgesehen ist.

Im Gegensatz zu den bekannten objektiven Verfahren, nimmt die Erfindung die Beobachtung im Dunkelfeld des alten subjektiven Verfahrens auf. Da die Teilchen in der Flüssigkeit nicht selbst leuchten und auch nicht nachleuchten, mußte ein Möglichkeit geschaffen werden, das von der Kamera aufgenommene Untergrundbild möglichst dunkel zu gestalten, dennoch aber bei Anwesenheit von Teilchen eine punktuelle Aufhellung zu erreichen. Dies wird durch zwei - gegebenenfalls unter Berücksichtigung der optischen Aktivität der Flüssigkeit selbst - unterschiedlich eingestellte Polarisatoren erreicht, die also derart eingestellt werden, daß ihre Polarisationsrichtung (bei nichtoptischer Flüssigkeit) senkrecht zueinander stehen und damit der Hintergrund weitgehend abgedunkelt wird. Die Fremdteilchen in Flüssigkeiten streuen nicht nur das Licht, sondern heben dabei auch die durch den ersten Polarisator vorgenommene Polarisation des Lichts weitgehend auf, bewirken also eine Depolarisation, so daß die Fernsehkamera durch das am Teilchen gestreute Licht einen intensiven Lichtfleck feststellen kann. Die größte Streuhelligkeit und die beste monotone Abhängigkeit der Streulichtintensität von der Teilchengröße ergab sich für die hier interessierenden Teilchen (größer) in Vorwärtsstreurichtung, so daß daher optische Orte von Lichtquelle, Streuzelle und Kamera vorzugsweise fluchtend angeordnet werden sollten. Die Streuzelle, also eine zu untersuchende Flasche, sollte also auf der optischen Achse des einfallenden Lichts im Gegenlicht betrachtet werden. Um auch bei zylindrischen Flaschen, die eine Aufhellung auf der optischen Achse bewirken, mit hoher Verstärkung, vorzugsweise voller Aussteuerung der Kamera arbeiten zu können, sieht eine bevorzugte Ausgestaltung vor, daß der Bereich der optischen Achse des Lichtstrahls ausgeblendet wird bzw. daß bei einem das Fluid umfassenden Mantel mit zum Lichtstrahl hin konvexer Oberfläche zentral vor diesem zwischen demselben und der Lichtquelle oder der Kamera sich parallel zur Achse der Mantelfläche erstreckend eine Abdeckung vorgesehen ist.

Sehr geringe Winkelabweichungen sind allerdings unschädlich. Diese können bei üblicher Flüssigkeit bis zu maximal 10 Grad betragen, sollten aber nicht darüberliegen, da einerseits die Intensität des von interessierenden Teilchen gestreuten Lichts nachläßt, andererseits störendes Streulicht von Blasen, Glasfehlern etc. zunimmt. Wenn die Kamera bei den erwähnten zylindrischen Flaschen außerhalb des zentralen und die Flasche bewirkten Aufhellungsbereichs angeordnet wird, kann die mittlere Blende fortbleiben.

Gute Signale ergaben sich bei inkohärenten Licht. Dieses kann weißes oder farbiges Licht, gegebenenfalls durch Farbfilterung, sein. Im letzteren Fall ist eine Beeinflussung der Detektoren durch Fremdlicht, wie Tageslicht, weitgehend auszuschließen. Eine lichtdichte Kapselung des Systems kann dann - gegenüber der Verwendung weißen Lichts einer Glühlampe - entfallen. Ein Farbfilter könnte z. B. vor dem Objektiv der Kamera plaziert werden.

Wegen der polarisierten Laserstrahlung ist beim Einsatz eines Lasers als Lichtquelle kein Polarisator vor dem Fluid erforderlich. Ein Interferenzfilter vor dem Objektiv der Kamera kann die Störlichtempfindlichkeit stark reduzieren. Die hohe Intensität des Laserstrahles bewirkt ein gutes Signal/Rausch-Verhältnis. Neben einem He/Ne-Laser käme ein Argon-Ionenlaser oder ein YAG-Laser in Betracht.

Bisher wurde davon ausgegangen, daß zur Detektion störender Teilchen die Auflösung der Kamera im Bereich der Größe der interessierenden Teilchen liegen müßte (so typischerweise DE-OS 30 43 031). Dies führte dazu, daß einerseits nur eine geringe Querschnittsfläche - und damit lediglich Ampullen - nicht aber größere Flaschen untersucht werden konnten, andererseits der Auswerteumfang erheblich war und große Rechner sowie eine große Zeit benötigte. Demgegenüber sieht die Erfindung vor, daß die Intensität mehrerer Bildelemente zur Bildung einer Meßzone durch Summation oder Mittelung zusammengefaßt wird. Eine Meßzone weist dabei vorzugsweise in der Größenordnung von 30 x 30 Bildelemente einer üblichen Fernsehkamera auf. Hierdurch wird eine Vereinfachung der Weiterverarbeitung der gemessenen Intensität erreicht, ohne daß ein Informationsverlust zu befürchten ist.

Die Kamera sieht nämlich das Streulicht nicht nur auf einem Bildelement, sondern aufgrund von Streueffekten, Unschärfen und vorzugsweise vorzunehmender maximaler Aussteuerung der Kamera, wo über mehrere Bildelemente der Fernsehkamera eine erhöhte Lichtintensität auch bei kleinen Teilchen festgestellt wird. Dies ermöglicht eine Echtzeitverarbeitung mit einem preiswerten Rechner wie insbesondere einem Standard-Tischrechner als Personalcomputer.

Da an freien Oberflächen des Fluids, insbesondere der Flüssigkeit, allgemein an Grenzflächen zwischen zwei Fluiden (seien sie Flüssigkeiten oder Gase), die Ober- oder Grenzfläche sich bewegen kann, was zu störenden Reflektionen und Fehlmessungen führen kann, ist in weiterer Ausgestaltung vorgesehen, daß die freie Oberfläche des Fluids aus dem Lichtstrahl ausgeblendet wird bzw. daß vor der Oberfläche des Fluids eine Abdeckung angeordnet ist.

Um auch Störungen aus sonstigen Randbereichen, insbesondere Grenzflächen zu umgebenden Flaschenmänteln, Rohrmänteln oder dergleichen zu vermeiden, die allerdings weniger kritisch sind, sehen weitere Ausgestaltungen vor, daß Randbereiche des Fluids ausgeblendet werden bzw. daß vor der beleuchteten Fläche des Fluids eine deren Randbereich abdeckende Maske angeordnet ist.

Die Ausblendung gewisser Randbereiche durch Masken oder Blenden ist unkritisch, soweit sie von ihrer Fläche her tatsächlich vergleichsweise klein sind, da die Fremdteilchen sich in den Fluiden bewegen und die Meßzeit jeweils so gewählt werden kann, daß ein zu erfassendes, sich bewegendes Fremdteilchen auf jeden Fall festgestellt wird. Eine Meßzeit von 1 Sek. ist hier durchaus ausreichend. Dies gilt insbesondere dann, wenn das Fluid in Bewegung, vorzugsweise Rotation versetzt wird, wobei dies insbesondere dadurch geschehen kann, daß ein Drehteller vorgesehen ist, um die Flüssigkeit in Bewegung zu setzen.

Eine Weiterbildung sieht vor, daß das Fluid mit gesteuert sich erhöhender Geschwindigkeit in Bewegung gesetzt wird bzw. daß der Drehteller mittels einer Steuereinrichtung derart in Bewegung setzbar ist, daß sich seine Geschwindigkeit kontinuierlich und stetig ändert. Durch diese Ausgestaltung wird erreicht, daß auch Fremdteilchen, die sich auf dem Boden eines Gefäßes abgesetzt haben, "aufgewirbelt" werden und durch ihre Bewegung festgestellt werden können.

Im Gegensatz zu Ampullen tritt aber bei größeren Behältnissen wie bei Infusionsflaschen bei der üblich horizontalen Zuführung der Flaschen zur Meßzelle ein erhebliches "Schwappen" der Flüssigkeit auf, das, wenn die hierdurch bedingten störenden Lichtreflexe vermieden werden sollen, die Abschirmung eines beträchtlichen Bereichs erfordert, so daß dann wiederum sich die Gefahr erhöht, daß relevante Fremdteilchen hinter der Blende bleiben und nicht erfaßt werden. Zur Lösung dieses Problems wird gemäß einem weiteren wichtigen Aspekt der Erfindung vorgesehen, daß die Behältnisse der Meßzelle vertikal zugeführt werden. Um weiterhin die Messung von Vibrationen anderer Einrichtungen, wie Förderern freizuhalten, ist vorgesehen, daß die Meßvorrichtung von sonstigen Einrichtungen, wie eben den Förderern getrennt und entkoppelt ist, sie insbesondere keine gemeinsamen Rahmen, Gestelle od. dgl. aufweisen, sondern ausschließlich über den gemeinsamen Standboden verbunden sind. Die zu untersuchenden Flaschen werden zur Messung durch einen Förderer Halteelementen der Meßeinrichtung übergeben und dann vom Förderer freigegeben, so daß sie ebenfalls nicht mehr in Verbindung stehen. Der Förderer kann dabei ein Paternoster sein, der die Flasche vorzugsweise am Hals unter dem Verschluß mittels einer Gabel ergreift. In der Meßzelle können die Flaschen auf einer Ablage abgestellt werden, werden vorzugsweise aber von zwei Bechern ergriffen, die gleichzeitig vertikale Randblenden bilden können. Auf jeden Fall löst sich die Paternostergabel vom Flaschenkopf und gibt diesen dann der Meßzelle frei, so daß die Messung durchgeführt werden kann.

Erfindungsgemäß ist vorgesehen, daß mindestens jeweils zwei Abtastungen jeder Meßzone vorgenommen werden, daß die Intensität für jede Meßzone gespeichert, die Intensität einer späteren Abtastung mit der gespeicherten Intensität für jede Meßzone verglichen und daß bei Abweichung der Intensitätsdifferenz über einen vorgegebenen Schwellwert ein Ausgangssignal erzeugt wird. Ein derartiges Verfahren ist an sich bekannt (EP-A-86 143), wobei aber unpolarisiertes Licht von unten gegen den Boden von Ampullen gerichtet und unter rechtem Winkel zur optischen Achse des auf die Ampullen fallenden Lichts betrachtet wird. Eine hinreichende Diskriminierung der bei vorliegender Erfindung interessierenden Teilchengrößen ist bei diesem Stand der Technik nicht möglich. Auch werden hier die Intensitätswerte der einzelnen Bildelemente bearbeitet, was einen erheblichen zeitlichen und konstruktiven Aufwand erfordert und ebenfalls nur bestenfalls die Prüfung kleiner Ampullen ermöglicht.

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnung im einzelnen erläutert wird. Dabei zeigt:
- Figur 1: Eine schematische Darstellung des Aufbaus der erfindungsgemäßen Vorrichtung;
- Figur 2: in Draufsicht eine andere Haltung einer zu untersuchenden Flasche in einer Meßzelle, und
- Figur 3: ein Blockschaltbild zum Ablauf der Auswertung in der Bild-Auswerteeinheit.

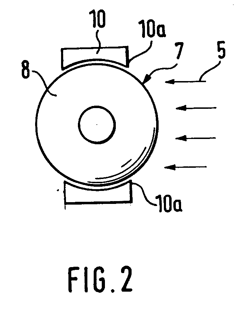

Die erfindungsgemäße Vorrichtung 1 weist eine Lichtquelle 2 in Form einer Glühlampe auf. Dieser kann ein Linsensystem 3 zur Aufweitung und Parallelisierung des Lichts nachgeordnet sein. Umlenkspiegel 3' gewährleisten eine kompakte Ausführung der Vorrichtung. Ein Fluid, insbesondere eine Flüssigkeit wird durch eine Meßzelle 4 hindurchgeführt oder in diese eingebracht. Im dargestellten Ausführungsbeispiel weist die Meßzelle 4 einen Drehteller 6 auf, auf den Infusionsflaschen 7 mit einer Infusionslösung 8 aufgestellt, in Rotation gesetzt und anschließend wieder angehalten werden, woraufhin die Messung bei rotierender Flüssigkeit 8 durchgeführt wird. Statt dessen können die Flaschen 7 auch auf einer räumlich getrennt von der Meßzelle 4 vorgegebenen Rotationseinrichtung gedreht und damit die Flüssigkeit 8 in der Flasche 7 in Rotation versetzt und anschließend die Flaschen 7 in die Meßzelle 4 eingebracht werden. In beiden Fällen können die Flaschen 7 zur Messung durch austauschbare Adapter 5 am Flaschenhals oder durch Klemmbecken 10 am Hauptteil (Figur 2) gehalten werden, wobei die Klemmbecken 10 seitliche Blenden 10a bilden. Zusammen mit - weiter unten im Detail beschriebenen - ebenfalls austauschbaren Masken 9 kann eine Anpassung an unterschiedliche Flaschen 7 vorgenommen werden. Weiterhin könnte durch die Meßzelle 4 eine Flüssigkeitsströmung geführt werden, beispielsweise in einem Rohr oder einem Kanal, der auf der der Lichtquelle 2 zugewandten und der dieser abgewandten Seite transparente Fenster aufweist.

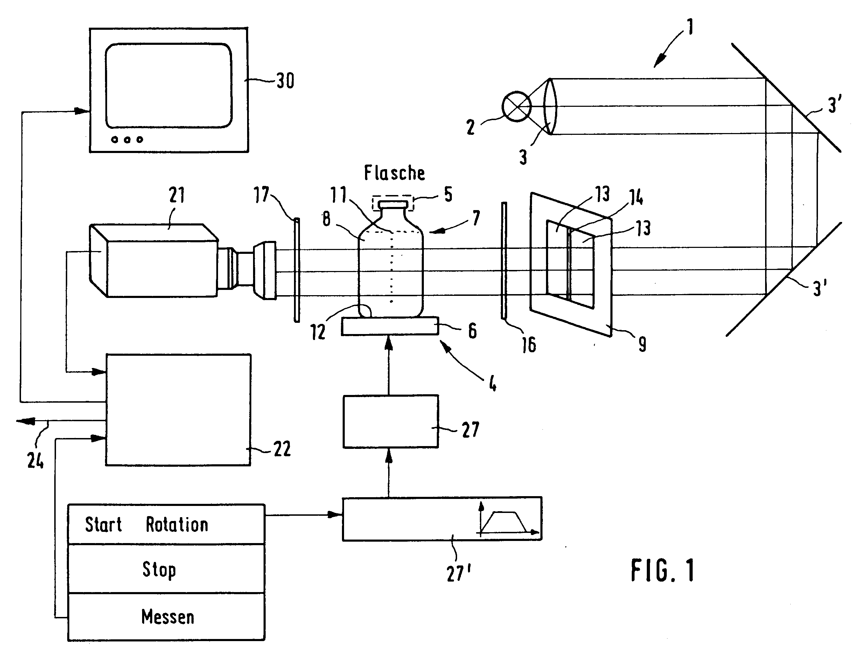

Im dargestellten Ausführungsbeispiel ist vor der Meßzelle 4 eine Maske 9 - die lediglich zur Darstellung perspektivisch gezeichnet ist, tatsächlich aber senkrecht zur Strahlrichtung bzw. zur Verbindungslinie Glühlampe 2 - Kamera 21 steht - angeordnet, die einen Teil des Lichtstrahls ausblendet bzw. einen Teil der Flasche 7 abdeckt. Dies gilt insbesondere für den Bereich der Oberfläche 11 der Flüssigkeit 8 in der Flasche 7, da durch Bewegungen der Oberfläche 11 Reflektionen in die Flüssigkeit 8 hinein erfolgen würden, die durch weitere Reflektion zu einer Störung der Messung führen könnten. Gegebenenfalls kann auch der Bodenbereich 12 der Flasche 7 abgedeckt sein. Bei zylindrischen Flaschen 7, wie eine solche in der Figur 1 dargestellt ist, hat sich herausgestellt, daß bei sonst gleichmäßiger Ausleuchtung der Flasche 7 bei Beobachtung im wesentlichen in Richtung der optischen Achse des einfallenden Lichts auf der Verbindungslinie, auf dieser also zwischen Glühlampe 2 und Achse der Flasche 7 parallel zu letzterer ein heller überbelichteter Streifen auftritt. Aus diesem Grunde ist die Maske 9 in zwei Fenster 13 geteilt, die durch einen mittleren Abdeckstreifen 14 voneinander getrennt sind. Beidseitig der Meßzelle 4 senkrecht zur Richtung des Lichtstrahls vor der Meßzelle 4 ist ein erster Polarisator 16 - auf den die Maske 9 aufgebracht sein kann - und hinter der Meßzelle 4 ein weiterer als Analysator dienender Polarisator 17 angeordnet. Dieser ist so eingestellt, daß, gegebenenfalls unter Berücksichtigung einer optisch aktiven Flüssigkeit 8 in der Flasche 7, die Intensität des durch den Analysator 17 hindurchtretenden Lichts minimal ist. Hinter dem Analysator 17 ist in Richtung des Lichtstrahls relativ zur Meßzelle 4 diametral der Glühlampe 2 gegenüberliegend eine Fernsehkamera 21 vorgesehen, die mit einer Bildauswerteeinheit 22 verbunden ist. An diese ist ein Monitor 30 angeschlossen. Die Bildauswerteeinheit 22 weist einen Ausgang 24 auf, über den sie ein Signal, insbesondere ein Steuersignal für eine Selektionsweiche zum Aussortieren fehlerhafter Flaschen 7 abgibt. Bei der dargestellten Ausführungsform mit einem unterhalb der Meßzelle 4 angeordneten Drehteller 6, auf dem eine Flasche 7 aufgestellt wird, ist eine Ablaufsteuerung 27 vorgesehen, die einerseits über eine Antriebssteuerung 27', einen Motor 27 des Drehtellers 6 und andererseits die Bildauswerteeinheit 22 steuert.

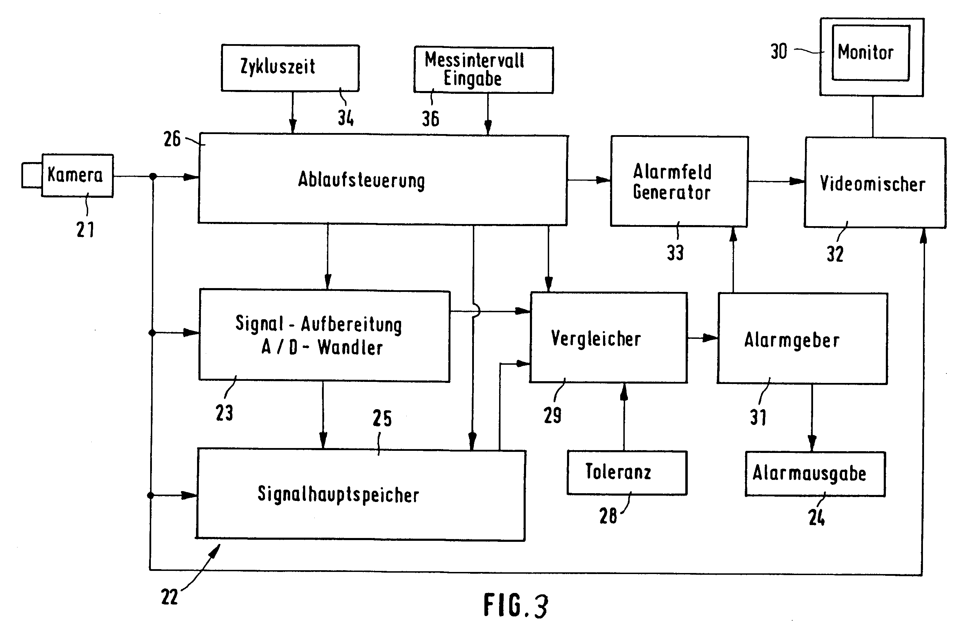

In Figur 3 ist ein Blockschaltbild zum Ablauf der Auswertung in der Bildauswerteeinheit 22 dargestellt. Die Bildauswerteeinheit 22 weist einen Signal-Aufbereiter 23 mit einem Analog-Digital-Wandler auf. Das aufgenommene Signal des einzelnen Bildelements der Kamera 21 wird über eine größere Meßzone von beispielsweise 30 x 30 Bildelementen durch Summation oder Mittelung zusammengefaßt und weiterverarbeitet. Dem Signal-Aufbereiter 23 ist einerseits ein Signal-Hauptspeicher 25 und andererseits ein Vergleicher 29 nachgeordnet, der ebenfalls einen Eingang für einen Ausgang des Signal-Hauptspeichers 25 aufweist. Weiterhin ist dem Vergleicher 29 ein Toleranzgeber oder Schwellwertsetzer 28 zugeordnet. Dem Vergleicher 29 folgt ein Alarmgeber 31, von dem einerseits eine Alarmausgabeeinheit 24, von der beispielsweise die Weichen im Förderweg der Flaschen 7 durch ein Steuersignal gesteuert werden, angesteuert wird. Das Signal der Kamera 21 wird weiterhin zu einem Videomischer 32 geführt, dem andererseits über einen Alarmfeldgenerator 33 ebenfalls ein Signal des Alarmgebers 31 zugeführt wird. Der Ablaufsteuereinheit 26 kann die Zykluszeit über einen Eingang 34 und das gewünschte Meßintervall über eine Eingabe 36 vorgegeben werden.

Die Flasche 7 wird durch eine Ablaufsteuerung 26 für eine vorgegebene Zeit über einen Motor 27 in Rotation versetzt, wobei vorzugsweise die Rotation mit gesteuert ansteigender und später gesteuert kontinuierlich abfallender Geschwindigkeit erfolgt, so daß ruckartige Bewegungen und Erschütterungen vermieden werden. Hierdurch werden Teilchen, die sich auf dem Boden 12 der Flasche 7 abgesetzt haben, auf und durch die Flüssigkeit 8 gewirbelt, so daß sie in ihrer Bewegung festgestellt werden. Nach Beendigung der Rotation und gegebenenfalls einer gewissen Wartezeit zur Beruhigung der Flüssigkeitsbewegung, die sich auch nach Stillstand des Drehtellers 6 und der Flasche 7 in dieser weiter dreht, werden über ein vorgegebenes Meßintervall Messungen vorgenommen, d. h. die Bildauswerteeinheit 22 aktiviert.

Das Signal der Kamera 21 wird zunächst über den Videomischer 32 dem Monitor 30 zugeführt, so daß dort jeweils das Bild der geprüften Flasche 7 permanent beobachtet werden kann.

Das aufgenommene Signal des einzelnen Bildelements der Kamera 21 wird während eines Meßintervalls dem Signal-Aufbereiter 23 zugeführt und in diesem digitalisiert. Das Digitalsignal wird dem Signal-Hauptspeicher 25 zugeführt und dort gespeichert. Weiterhin wird das Digitalsignal zeitlich mehrfach, z. B. alle 50 ms dem Vergleicher 29 zugeführt, dem ebenfalls durch die Ablaufsteuereinheit 26 das im Signal-Hauptspeicher 25 gespeicherte Signal zugeführt wird. Im Vergleicher 29 werden die zu verschiedenen Zeiten erfaßten Signale gleichbleibender Meßzonen für die einzelnen Meßzonen verglichen und festgestellt, ob die mittels eines Differenzverstärkers erhaltenen Signaldifferenzen über einen extern vorgegebenen Schwellwert, der vom Schwellwertgeber 28 dem Vergleicher 29 zugeführt wird, abweichen. Wenn der Schwellwert überschritten wird, gibt die Bildauswerteeinheit 22 einerseits ein Signal über eine Alarmausgabe 24 ab. Es kann sich hierbei um ein akkustisches oder optisches Signal handeln, das angibt, daß die gerade geprüfte Flasche 7 nicht ordnungsgemäß und daher auszuscheiden ist. Bei Automatikzug der fortgeführten Flaschen 7 kann es sich um ein Steuersignal handeln, welches eine Weiche betätigt, durch welche die gerade überprüfte, fehlerhafte Flasche 7 aus dem Förderweg der als in Ordnung befundenen Flaschen abgezweigt wird. Andererseits wird in der dargestellten bevorzugten Ausführungsform das Signal einem Alarmfeldgenerator 33 zugeführt, der in der fraglichen Meßzone über den Videomischer 32 eine Markierung auf dem Monitor 30 anzeigt, wodurch angegeben wird, wo ein Fremdteilchen festgestellt wurde.

Es hat sich herausgestellt, daß bei Auftreten größerer Fremdkörper in Flüssigkeiten 8, insbesondere bei hoher Aussteuerung diese punktuell eine erhebliche Aufhellung im Kamerabild bedingen, welche durch die Bildauswerteeinheit 22 festgestellt und in der beschriebenen Weise verarbeitet wird. Diese Aushellung kann deutlich am Monitor 30 festgestellt werden. Es hat sich weiterhin herausgestellt, daß die Aufhellung bei wesentlich größeren Teilchen größer ist als bei kleineren Teilchen (zumindest gleiche Teilchenart vorausgesetzt), so daß hierdurch eine Selektion der Flaschen 7 nach Größe der in diesen festgestellten Fremdkörpern vorgenommen werden kann. Insbesondere ist vorgesehen, daß ein Schwellwert derart gesetzt wird, daß Teilchen, die beispielsweise größer als 50 Mikrometer (unter Berücksichtigung von Toleranzen) sind, zu einem Steuersignal über die Alarmausgabeeinheit 24 führen, kleinere Teilchen aber nicht. Hiermit ist beispielsweise für Infusionslösungen 8 die Grenze so niedrig gesetzt, daß als gut befundene Infusionsflaschen 7 keine Teilchen enthalten, die zu einer Verstopfung der Infusionsnadel oder Gefährdung des Patienten hinsichtlich Thrombosen führen können, was vermieden werden soll, andererseits aber die Größe auch nicht so niedrig gesetzt, daß eine zu große Anzahl von Flaschen 7 ausgeschieden würden, obwohl diese bestenfalls Teilchen enthalten, die unkritisch sind. Es hat sich gezeigt, daß die erfindungsgemäße Vorrichtung eine Selektion mit hinreichender Genauigkeit ermöglicht.

Bei punktueller Intensitätserhöhung durch ein Fremdteilchen in einer Flüssigkeit 8 bei Durchführung des erfindungsgemäßen Verfahrens wird die erhöhte Intensität nicht nur in einem Bildelement oder nur in einer der Größe des Teilchens entsprechenden, vom Elektronenstrahl abgetasteten Fläche der Aufnahmeröhre wesentlich erhöht, sondern in einem größeren Bereich mehrerer Bildelemente einer herkömmlichen Fernsehkamera 21. Entsprechendes gilt bei einer Kamera mit CCDs, wobei aber ein Vidicon, insbesondere ein Z-Vidicon oder Novicon vor allem bei größeren Flaschen 8 bevorzugt wird, da es eine vielfach höhere Empfindlichkeit als eine CCD-Kamera ermöglicht.

Es ist daher, wie gesagt, in erfindungsgemäßer Ausgestaltung der Bildauswerteeinheit 22 vorgesehen, daß die einzelnen Bildelemente der Fernsehkamera 21 zu größeren Auswertebereichen zusammengefaßt werden, die beispielsweise 30 x 30 Bildelemente aufweisen und innerhalb derer die Intensitäten der einzelnen Bildelemente durch Summation oder Mittelung zusammengefaßt und weiterverarbeitet werden. Dies trägt zur Vereinfachung der weiteren Verarbeitung und zur Reduzierung des Verarbeitungsaufwandes bei.

## Patentansprüche

1. Verfahren zum Feststellen von Fremdkörpern in Fluiden, insbesondere in in Behältern enthaltenen Flüssigkeiten, wie Infusionslösung od. dgl., wobei das Fluid flächig mit polarisiertem Licht beleuchtet und das durch das Fluid hindurchtretende Licht im wesentlichen in Richtung der optischen Achse des einfallenden Lichts durch einen zu dessen Polarisationsrichtung gekreuzten Analysator hindurch betrachtet wird, wodurch das durch das Fluid hindurch tretende Licht weitgehend abgedunkelt ist, dadurch gekennzeichnet, daß während der Messung der Behälter ruht und die Flüssigkeit in Bewegung ist, daß die Lichtintensität einzelner Bildelemente mittels optoelektrischer Wandlung gemessen wird, daß die Intensitäten mehrerer Bildelemente in einer durch diese gebildeten Meßzone durch Summation oder Mittelung zusammengefaßt werden, daß zeitlich nacheinander mindestens zwei Messungen jeder Meßzone vorgenommen werden und diese für jede Meßzone miteinander verglichen werden und daß bei Abweichung der Intensitätsdifferenz über einen vorgegebenen Schwellwert ein Ausgangssignal erzeugt wird.

2. Verfahren nach Anspruch 1, dadurch gekennzeichnet, daß das Fluid vor der Messung ausschließlich in vertikaler Richtung gefördert wird.

3. Verfahren nach Anspruch 1 oder 2, dadurch gekennzeichnet, daß bei einem das Fluid umfassenden Mantel mit zum Lichtstrahl hin konvexer Oberfläche die Mitte des Lichtstrahls ausgeblendet wird.

4. Verfahren nach einem der Ansprüche 1 bis 3, dadurch gekennzeichnet, daß die freie Oberfläche des Fluids aus dem Lichtstrahl ausgeblendet wird.

5. Verfahren nach einem der Ansprüche 1 bis 4, dadurch gekennzeichnet, daß Randbereiche des Fluids ausgeblendet werden.

6. Verfahren nach einem der vorangehenden Ansprüche, dadurch gekennzeichnet, daß das Fluid mit weißem Licht beleuchtet wird.

7. Verfahren nach einem der Ansprüche 1 bis 6, dadurch gekennzeichnet, daß der Analysator automatisch auf maximale Abdunkelung nachgeführt wird.

8. Verfahren nach einem der vorangehenden Ansprüche, dadurch gekennzeichnet, daß das Fluid in Rotation versetzt wird.

9. Verfahren nach Anspruch 8, dadurch gekennzeichnet, daß das Fluid mit gesteuert sich erhöhender Geschwindigkeit in Bewegung gesetzt wird.

10. Verfahren nach einem der vorangehenden Ansprüche, dadurch gekennzeichnet, daß ein zu messendes Objekt in eine Meßzelle eingebracht, eine kurzzeitige Messung durchgeführt und das Objekt weitergefördert wird.

11. Vorrichtung zum Feststellen von Fremdköpern in Fluiden, insbesondere von in Behältern (7) enthaltenen Flüssigkeiten, wie Infusionslösungen (8) od. dgl., mit einer Lichtquelle (2, 3, 16), die das Fluid flächig mit polarisiertem Licht beleuchtet und mit einem im wesentlichen auf der der Lichtquelle (2, 3, 16) abgewandten Seite angeordneten, auf Dunkelfeldbeobachtung eingestellten Analysator (17), dadurch gekennzeichnet, daß während der Messung der Behälter ruht und die Flüssigkeit in Bewegung ist, daß im wesentlichen auf der optischen Achse des auf das Fluid fallenden Lichts eine Fernsehkamera als Detektionseinrichtung (21) mit in Form einer Matrix angeordneten Bildelementen angeordnet ist, der eine Auswerteeinheit (22) nachgeordnet ist, die eine Einrichtung (23) zum Zusammenfassen der Intensitäten von mehreren einzelnen Bildelementen einer Meßzone zu einem Meßwert durch Summation oder Mittelung aufweist, und daß ein Vergleicher (29) mit einer einstellbaren Schwelle zum Vergleichen der Differenz von zu verschiedenen Zeiten erfaßten, zusammengefaßten Meßwerten einer Meßzone mit der Schwelle vorgesehen ist.

12. Vorrichtung nach Anspruch 11, dadurch gekennzeichnet, daß ein die Flüssigkeit in Behältnissen in vertikaler Richtung der Meßzelle (4) zuführender Förderer vorgesehen ist.

13. Vorrichtung nach Anspruch 11 oder 12, dadurch gekennzeichnet, daß bei einer das Fluid (8) umfassenden zylindrischen Mantelfläche (7) zentral zu dieser zwischen derselben und der Lichtquelle (2), sich parallel zur Achse der Mantelfläche erstreckend, eine Abdeckung (14) vorgesehen ist.

14. Vorrichtung nach einem der Ansprüche 11 bis 13, dadurch gekennzeichnet, daß die Lichtquelle eine Quelle inkohärenten Lichts, wie eine Glühlampe od. dgl., ist und daß vor dem Fluid ein Polarisator (16) angeordnet ist.

15. Vorrichtung nach einem der Ansprüche 11 bis 14, dadurch gekennzeichnet, daß vor der Oberfläche (11) des Fluids (8) eine Abdeckung angeordnet ist, durch die die freie Oberfläche des Fluids aus dem Lichtstrahl ausgeblendet wird.

16. Vorrichtung nach einem der Ansprüche 11 oder 15, dadurch gekennzeichnet, daß vor der beleuchteten Fläche des Fluids (8) eine deren Randbereich ausblendende Maske (13) angeordnet ist.

17. Vorrichtung nach einem der Ansprüche 11 bis 16, dadurch gekennzeichnet, daß ein Drehteller (6) vorgesehen ist, um die Flüssigkeit (8) in Bewegung zu setzen.

18. Vorrichtung nach Anspruch 17, dadurch gekennzeichnet, daß der Drehteller (6) mittels einer Ablaufsteuerung (26) derart in Bewegung setzbar ist, daß sich seine Geschwindigkeit kontinuierlich und stetig ändert.

## Claims

1. Method for detecting impurities in fluids, particularly in liquid contained in containers, such as an infusion solution or the like, the fluid being illuminated flat with polarized light and the light passing through the fluid is observed substantially in the direction of the optical axis of the incident light through an analyzer crossing the polarization direction thereof, so that the light passing through the fluid is substantially darkened, characterized in that during the measurement the container is stationary and the liquid is moving, that the light intensity of individual image elements is measured by means of optoelectric conversion, that the intensities of several image elements in a measuring zone formed by the latter are combined by summation or averaging, that in time succeeding manner at least two measurements of each measuring zone are carried out and are compared with one another for each measuring zone and that in the case of a variation of the intensity difference over and beyond a predetermined threshold an output signal is produced.

2. Method according to claim 1, characterized in that, prior to the measurement, the fluid is exclusively delivered in the vertical direction.

3. Method according to claim 1 or 2, characterized in that in the case of a jacket surrounding the fluid with a surface convex to the light beam, the centre of the light beam is masked out.

4. Method according to one of the claims 1 to 3, characterized in that the free surface of the fluid is masked out from the light beam.

5. Method according to one of the claims 1 to 4, characterized in that marginal areas of the fluid are masked out.

6. Method according to one of the preceding claims, characterized in that the fluid is illuminated with white light.

7. Method according to one of the claims 1 to 6, characterized in that the analyzer is automatically adjusted to maximum darkening.

8. Method according to one of the preceding claims, characterized in that the fluid is rotated.

9. Method according to claim 8, characterized in that the fluid is moved with a speed increasing in controlled manner.

10. Method according to one of the preceding claims, characterized in that an object to be measured is introduced into a measuring cell, a brief measurement is performed and the object is conveyed on.

11. Device for detecting impurities in fluids, particularly in liquids contained in containers (7), such as infusion solutions (8) or the like, with a light source, which illuminates the fluid flat with polarized light and with an analyzer (17) set to dark sealed observation and arranged substantially on the side remote from the light source (2, 3, 16), characterized in that during the measurement the container is stationary and the liquid moving, that substantially on the optical axis of the light incident on the fluid a television camera is arranged as the detecting means (21) with image elements arranged in the form of an array and is followed by an evaluating unit (22), which has a device (23) for combining the intensities of several individual image elements of a measuring zone to form a single measured value by summation or averaging and that a comparator (29) with an adjustable threshold is provided for comparing the difference of combined measured values of a measuring zone detected at different times with the threshold.

12. Device according to claim 11, characterized in that a conveyor supplying the liquid in containers in the vertical direction of the measuring cell (4) is provided.

13. Device according to claim 11 or 12, characterized in that in the case of a cylindrical circumferential surface (7) surrounding the fluid (8) there is provided centrally to the latter between it and the light source (2) a cover (14) extending parallel to the axis of the circumferential surface.

14. Device according to one of the claims 11 to 13, characterized in that the light source is a source of incoherent light, such as a bulb or the like and that a polarizer (16) is positioned upstream of the fluid.

15. Device according to one of the claims 11 to 14, characterized in that upstream of the surface (11) of the fluid (8) is provided a cover, which masks out from the light beam the free surface of the fluid.

16. Device according to one of the claims 11 or 15, characterized in that upstream of the illuminated surface of the fluid (8) is provided a mask (13) masking out the marginal area thereof.

17. Device according to one of the claims 11 to 16, characterized in that a rotary table (6) is provided in order to move the liquid (8).

18. Device according to claim 17, characterized in that the rotary table (6) can be set into movement by means of a sequence control (26) in such a way that its speed continuously and smoothly varies.

## Revendications

1. Procédé de détection de corps étrangers dans des fluides, en particulier dans des liquides contenus dans des récipients, tels que solution de perfusion ou liquide analogue, dans lequel le fluide est éclairé de manière uniforme avec de la lumière polarisée et la lumière traversant le fluide est observée de manière générale dans la direction de l'axe optique de la lumière incidente à travers un analyseur croisé par rapport à la direction de polarisation de celle-ci, de sorte que la lumière traversant le fluide est très obscurcie, caractérisé par le fait que pendant la mesure, le récipient est immobile et le liquide est en mouvement, que l'intensité lumineuse d'éléments d'image individuels est mesurée par conversion optoélectrique, que les intensités de plusieurs éléments d'image sont, dans une zone de mesure formée par ceux-ci, groupées par sommation ou formation de la moyenne, qu'au moins deux mesures de chaque zone de mesure sont faites successivement et comparées, et que si la différence des intensités dépasse un seuil fixé, il est produit un signal de sortie.

2. Procédé selon la revendication 1, caractérisé par le fait qu'avant la mesure, le fluide est transporté uniquement dans la direction verticale.

3. Procédé selon l'une des revendications 1 et 2, caractérisé par le fait que si le fluide est entouré d'une enveloppe à surface convexe vers le faisceau lumineux, le milieu de celui-ci est éliminé.

4. Procédé selon l'une des revendications 1 à 3, caractérisé par le fait que la surface libre du fluide est mise hors du faisceau lumineux.

5. Procédé selon l'une des revendications 1 à 4, caractérisé par le fait que des zones de bord du fluide sont mises hors du faisceau lumineux.

6. Procédé selon l'une des revendications précédentes, caractérisé par le fait que le fluide est éclairé avec de la lumière blanche.

7. Procédé selon l'une des revendications 1 à 6, caractérisé par le fait que l'analyseur est réglé automatiquement à l'obscurcissement maximal.

8. Procédé selon l'une des revendications précédentes, caractérisé par le fait que le fluide est mis en rotation.

9. Procédé selon la revendication 8, caractérisé par le fait que le fluide est mis en mouvement à une vitesse croissant de façon commandée.

10. Procédé selon l'une des revendications précédentes, caractérisé par le fait qu'un objet à mesurer est mis dans une cellule de mesure, une mesure de courte durée est effectuée et l'objet est évacué.

11. Dispositif de détection de corps étrangers dans des fluides, en particulier des liquides contenus dans des récipients (7), tels que solutions de perfusion (8) ou liquides analogues, comportant une source lumineuse (2, 3, 16) qui éclaire le fluide de manière uniforme avec de la lumière polarisée et un analyseur (17) placé de manière générale sur le côté opposé à cette source lumineuse (2, 3, 16) et réglé pour l'observation sur fond noir, caractérisé par le fait que pendant la mesure, le récipient est immobile et le liquide est en mouvement, que de manière générale sur l'axe optique de la lumière tombant sur le fluide est placée une caméra de télévision servant d'appareil de détection (21) et ayant des éléments d'image disposés en une matrice, en aval de laquelle est prévu un appareil d'exploitation (22) qui présente un dispositif (23) de groupement en une valeur de mesure, par sommation ou formation de la moyenne, des intensités de plusieurs éléments d'image individuels d'une zone de mesure, et qu'il est prévu un comparateur (29) à seuil réglable pour la comparaison à ce seuil de la différence de valeurs mesurées groupées saisies à des instants différents.

12. Dispositif selon la revendication 11, caractérisé par le fait qu'il est prévu un transporteur amenant dans la direction verticale à la cellule de mesure (4) le liquide contenu dans des récipients.

13. Dispositif selon l'une des revendications 11 et 12, caractérisé par le fait que si le fluide (8) est enveloppé d'une surface cylindrique (7), un élément de masquage (14) s'étendant parallèlement à l'axe de cette surface est prévu au centre par rapport à celle-ci entre celle-ci et la source lumineuse (2).

14. Dispositif selon l'une des revendications 11 à 13, caractérisé par le fait que la source lumineuse est une source de lumière incohérente telle que lampe à incandescence ou analogue, et que devant le fluide est placé un polariseur (16).

15. Dispositif selon l'une des revendications 11 à 14, caractérisé par le fait que devant la surface (11) du fluide (8) est placé un élément de masquage qui met la surface libre du fluide hors du faisceau lumineux.

16. Dispositif selon l'une des revendications 11 à 15, caractérisé par le fait que devant la surface éclairée du fluide (8) est placé un masque (12) qui masque la zone de bord de celui-ci.

17. Dispositif selon l'une des revendications 11 à 16, caractérisé par le fait qu'il est prévu un plateau tournant (6) pour la mise en mouvement du liquide (8).

18. Dispositif selon la revendication 17, caractérisé par le fait que le plateau tournant (6) peut être mis en mouvement au moyen d'une commande de processus (26) de façon telle que sa vitesse varie de façon continue et en permanence.
